Europäisches Patentamt

European Patent Office

Office européen des brevets

(19)

(11) Publication number: **0 467 998 B1**

# EUROPEAN PATENT SPECIFICATION

(45) Date of publication of patent specification: **28.09.94**

(51) Int. Cl.[5]: **C07K 7/08**, C07K 7/10, C07K 7/00, C07K 17/00, G01N 33/574, G01N 33/531

(21) Application number: **90911368.0**

(22) Date of filing: **13.04.90**

(86) International application number: **PCT/US90/02017**

(87) International publication number: **WO 90/12807 (01.11.90 90/25)**

(54) **PEPTIDES FROM RETINOBLASTOMA GENE PRODUCTS AND ANTIBODIES THERETO.**

(30) Priority: **14.04.89 US 338289**
**11.04.90 US 508051**

(43) Date of publication of application:
**29.01.92 Bulletin 92/05**

(45) Publication of the grant of the patent:
**28.09.94 Bulletin 94/39**

(84) Designated Contracting States:
**AT BE CH DE DK ES FR GB IT LI LU NL SE**

(56) References cited:
**EP-A- 0 259 031**
**WO-A-87/06940**

**ONCOGENE RESEARCH, vol. 1, 1989, Harwood Academic Publishers GmbH, (GB); J.-Y. SHEW et al., pp. 205-214&NUM;**

(73) Proprietor: **BAYLOR COLLEGE OF MEDICINE**
**One Baylor Plaza**
**Houston, TX 77030 (US)**

(72) Inventor: **XU, Hong-Ji**
**10 Moonseed Place**
**The Woodlands, TX 77381 (US)**
Inventor: **BENEDICT, William, F.**
**21 E. Wedgewood Glen**
**The Woodlands, TX 77381 (US)**
Inventor: **HU, Shi-Xue**
**10 Moonseed Place**
**The Woodlands, TX 77381 (US)**

(74) Representative: **Lucas, Brian Ronald et al**
**Lucas & Co.**
**135 Westhall Road**
**Warlingham Surrey CR6 9HJ (GB)**

## Description

### Background of the Invention

Lack of genetic material normally present in humans has been linked to the development of tumors. The lack of the retinoblastoma (Rb) gene in humans has been linked to the development of retinoblastoma, the most common malignant ocular tumor in childhood. Benedict, W.F. "Recessive Human Cancer Susceptibility Genes (Retinoblastoma and Wilm's Loci)" Ad. Vir. Oncol., Vol. 7, Klein Ed., Raven Press 1987. The lack of the Rb gene has also been linked to the development of osteosarcomas, fibrosarcomas and other soft tissue sarcomas and has been implicated in other tumor development or progression such as small cell lung carcinoma and breast cancer.

The Rb gene is characterized as a "suppressor" or "regulatory" gene since its presence inhibits tumor development. Murphree, A. L. and Benedict, W. F. "Retinoblastoma: clues for human oncogenes", Science, Vol. 223, pp.1028-1033 (1984). Usually a gene contains a coding sequence for the production of certain proteins on the cellular level. The lack of the gene will be indicated by the lack of the protein coded by the gene provided the proper stimuli has been imported to the cell to activate the gene. In the case of a suppressor gene, such as the Rb gene, the detection of the presence or absence of the gene through the Rb gene products, i.e., proteins produced by Rb gene expression is a very important diagnostic tool and useful in therapy selection and genetic counseling.

The Rb gene (also known as the Rb susceptibility gene) is on chromosome 13, region 13g14. The human Rb gene cDNA sequence has been published. "A Method of Detecting the Predisposition to Retinoblastoma and a Method for Detecting a Retinoblastoma Gene Defect in Tumors Using a Retinoblastoma Gene Probe", Fung, T'Ang, Murphree and Benedict, Int'l. Publn. No. WO 88/09387, published December 1, 1988 (Int'l. Appln. No. PCT/US88/01809); Lee, W. H., Shew, J. Y., Hong, F. D., Sery, T. W., Donoso, L. A., Young, L. J., Bookstein, R., Lee, E. Y. H. "The retinoblastoma susceptibility gene encodes a nuclear phosphoprotein associated with DNA binding activity", Nature, Vol. 329, pp.642-645 (1987). Friend, S. H., Horowitz, J. M., Gerber, M. R., Wang, X. F. Bogenmann, E., Li, F. P., Weinberg, R. A. "Deletions of a DNA sequence in retinoblastoma and mesenchymal tumors: organization of the sequence and its encoded protein", Proc., Natl. Acad. Sci. U.S.A., Vol. 84, pp.9059-9063 (1987).

Gene probes which identify the presence of certain Rb gene DNA sequences have been developed. Further investigations report the loss of the Rb gene in one or both alleles of chromosome 13 in certain patients as well as the recessive nature of the gene. According to the Rb cDNA sequence, a protein product of 928 amino acids is expected. However, no native Rb protein is available, and no antibody specific to the Rb protein from normal cells has been finally authenticated.

Several antigens have been preliminarily identified as raising antibodies to the human Rb gene products. Lee et al., "The Retinoblastoma Susceptibility Gene Encodes a Nuclear Phosphoprotein Associated with DNA Binding Activity," 329 Nature 642-645. Yokota et al., "Altered Expression of the Retinoblastoma (Rb) Gene in Small-Cell Carcinoma of the Lung," 3 Oncogene 471-475. However, no antibody, when used with an immunohistochemical stain marker, has been documented to be able to distinguish known Rb positive from known Rb negative cells.

The development of antibodies highly specific to the regulatory or suppressor gene (Rb gene) products can be used to characterize a functional normal Rb protein synthesis and identify the presence of abnormal (truncated) Rb proteins. Identification of the truncated Rb proteins by a highly specific antibody can be used for pre or post-natal diagnosis.

### Summary of the Invention

The peptides of the invention are defined in claim 1.

A characterization of polypeptide antigens corresponding to epitopes in human retinoblastoma (Rb) gene products has been made including preparation of synthetic polypeptides. The synthetic polypeptides alone and in combination produce antisera with IgG antibodies specific to Rb gene products. A preferred synthetic antigenic polypeptide produces a highly specific, monospecific IgG to Rb proteins.

The Rb gene with its cDNA sequence is known. The longest open reading frame which is considered the cDNA sequences coding for expressed human Rb protein amino acid sequence has been published as cited in the "Background" section. Lee, W. H., Shew, J. Y., Hong, F. D., Sery, T. W., Donoso, L. A., Young, L. J., Bookstein, R., Lee, E. Y. H. "The retinoblastoma susceptibility gene encodes a nuclear phosphoprotein associated with DNA binding activity", Nature, Vol. 329, pp.642-645 (1987). Friend, S. H., Horowitz, J. M., Gerber, M. R., Wang, X. F. Bogenmann, E., Li, F. P., Weinberg, R. A. "Deletions of a DNA sequence in retinoblastoma and mesenchymal tumors: organization of the sequence and its encoded protein", Proc., Natl. Acad. Sci. U.S.A., Vol. 84, pp.9059-9063 (1987). However, there has been no native Rb protein available for immunogen preparation. The Rb protein is suspected to be conserved among the vertebrate species. Lee, W. H., Bookstein, R., Hong, F., Young, L. J., Shew, J. Y., Lee, Y. H. P. "Human retinoblastoma susceptibility gene: cloning, identification, and sequence", Science, Vol. 235, pp.1394-1399 (1987). The conservation may contribute to low immunogenicity. Therefore, the polypeptide antigens of this invention were developed without available native Rb protein, which protein in its natural state is considered difficult to immunize against.

The polypeptide sequences are selected from the deduced Rb protein amino acid sequence as described in Lee, W. H., Shew, J. Y., Hong, F. D., Sery, T. W., Donoso, L. A., Young, L. J., Bookstein, R., Lee, E. Y. H. "The retinoblastoma susceptibility gene encodes a nuclear phosphoprotein associated with DNA binding activity", Nature, Vol. 329, pp.642-645 (1987). Friend, S. H., Horowitz, J. M., Gerber, M. R., Wang, X. F. Bogenmann, E., Li, F. P., Weinberg, R. A. "Deletions of a DNA sequence in retinoblastoma and mesenchymal tumors: organization of the sequence and its encoded protein", Proc., Natl. Acad. Sci. U.S.A., Vol. 84, pp.9059-9063 (1987), which are incorporated by reference in the specification herein. Also, the pending patent application entitled "A Method of Detecting the Predisposition to Retinoblastoma and a Method for Detecting a Retinoblastoma Gene Defect in Tumors Using a Retinoblastoma Gene Probe" by Fung, T'Ang, Murphree and Benedict, Int'l. Publn. No. WO 88/09387, published December 1, 1988 (Int'l. Appln. No. PCT/US88/01809) contains the Rb cDNA sequence. The selection of the polypeptide antigens was based on unique criteria for amino acid sequences with a combination of low solubility and high hydrophilicity characteristics.

The low solubility criteria of the polypeptide sequences is contrary to accepted principles that high solubility is better for antigenic response. The balancing of the solubility characteristics of the amino acids along with the hydrophilicity resulted in a successful method for identifying synthetic antigens to the Rb gene product. The method selects peptides of 10 or more amino acids in desired sequences with low solubility and high hydrophilicity.

Low solubility of the polypeptide shall mean that in sequences of at least 10 amino acids selected from the amino acid sequence of the Rb protein, the number of grade I and grade II amino acids is greater than or equal to the number of Grade III amino acids; preferably, the number of grade I amino acids is greater or equal to the number of grade III amino acids. Grade I amino acids are insoluble, Grade II amino acids are slightly soluble and Grade III amino acids are soluble in water. (See TABLE 1.)

For the purpose of this disclosure, hydrophilicity value was determined by the method of Hopp, T. P. and Woods, K. R., "Prediction of protein antigenic determinants from amino acid sequences", Proc. Natl. Acad. Sci. U.S.A., Vol. 78, pp.3824-3828 (1981), which is incorporated by reference into this disclosure. (See TABLE 2.) The hydrophilicity value of the polypeptide is greater than 0.75, which is calculated by averaging of the individual amino acid hydrophilicity value as assigned by Hopp and Woods. The synthetic polypeptide antigen of this invention is defined as "flexible" since it must have low solubility characteristics as well as high hydrophilicity characteristics included in the amino acids comprising the sequence. The synthetic polypeptide can be used with or without a carrier protein.

This method of Rb antigen sequence identification has been used to identify at least six synthetic antigenic polypeptides. Among the six polypeptides, a preferred amino acid sequence has been shown to possess superior characteristics for production of highly specific antibodies to the Rb protein. An alternative embodiment includes a flexible polypeptide with a linking amino acid added to a terminal end capable of associating a carrier protein. Linking amino acids include cysteine, tyrosine, lysine, aspartic acid and glutamic acid. Other synthetic polypeptide antigens may be identified using the method of antigen sequence identification.

The antisera were prepared by immunizing rabbits using selective synthetic polypeptide antigens. In some cases, the antigen was not conjugated to a carrier protein. The resulting antisera were precipitated with ammonium sulfate and desalinized in the initial steps and then separated by DEAE chromatography to obtain the IgG fraction. The specific anti-Rb IgGs were separated from a peptide-CNBr-Sepharose column at a low pH range of 2.4-2.2. Pure high-affinity anti-Rb antibodies prepared using the peptides of this invention immunoprecipitated Rb proteins. The antibodies produced by this invention were used to identify a distinct immunoprecipitation and Western blot pattern for normal Rb proteins. The antibodies may be used to detect Rb proteins on the cellular or subcellular level.

The highly specific antibodies have been associated with markers for detection of Rb gene product. Immunohistochemcial staining procedures using the highly specific antibodies detect positive in known Rb positive cells and negative in known Rb negative cells. The staining procedure has been used for in vivo tissue analysis as well as cell culture. The highly specific antibodies of this invention have provided useful information on the subcellular location of the Rb protein during the cell cycle.

Since the absence, alteration or mutation of the Rb gene is implicated in various tumors, the production of a highly specific antibody to detect the presence or absence of the Rb protein is valuable as a screening technique. Further, it has been found that certain anti-Rb IgGs also detect abnormal Rb proteins with immunoprecipitation and Western blotting which can be used as a diagnostic tool for Rb gene irregularity when there is not a total absence of the Rb gene product from both Rb alleles.

The detection of abnormal Rb proteins allows for examination of predisposition to certain cancers on the protein level in the constitutional cells. The highly specific anti-Rb IgGs can be used in various diagnostic procedures generally utilizing purified antibodies. A biopsy tissue sample can be used for both immunohistochemical staining and Western blotting. These techniques are more practical and accepted diagnostic tools as opposed to mRNA and cDNA probe hybridization methods.

Brief Description of the Drawings

Fig. 1 is the deduced amino acid sequence for the normal human Rb protein and epitope location.

Fig. 2 is the genomic organization of the human Rb gene. Hind III sites are represented by vertical lines. Individual exons are shown as black boxes. Parentheses represent uncertainty in the size of the intron restriction fragments. SalI sites(S) are indicated. The exons are also shown as related to their positions along the Rb cDNA map.

Figs. 3a, 3b, 3c and 3d are a hydrophilicity profile of the deduced Rb protein.

Fig. 4 shows the results and corresponding schematic interpretation of Immunoprecipitation and Western Blotting of the normal human fibroblast Rb gene product.

Fig. 5 is a schematic representation of immunoprecipitation of certain shortened Rb proteins in constitutional cells and tumor cells.

Fig. 6 is a photograph of staining results on Rb negative and Rb positive cultured cells.

Detailed Description of the Invention

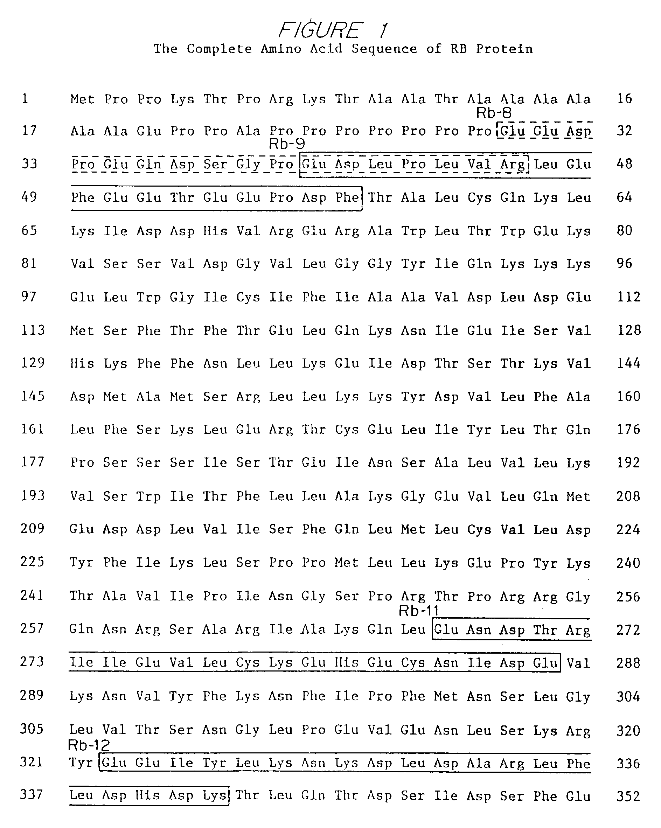

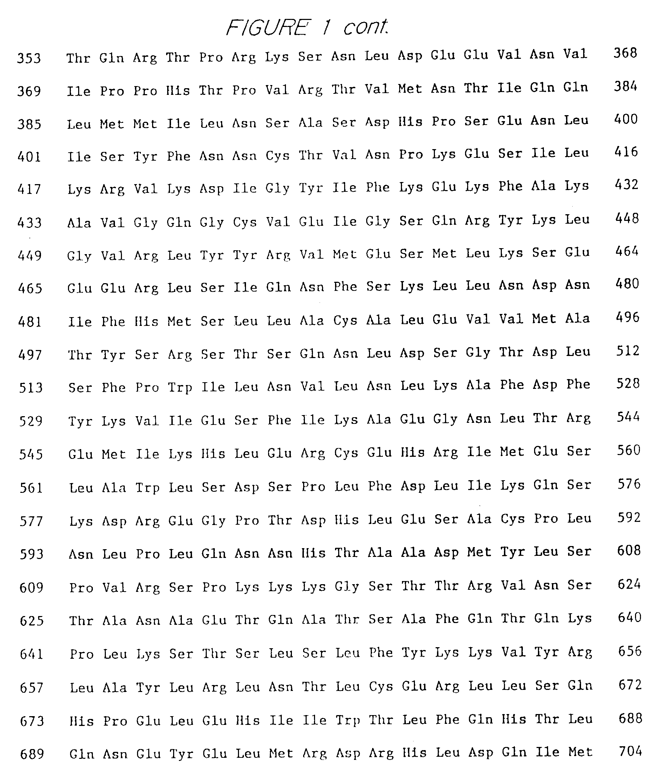

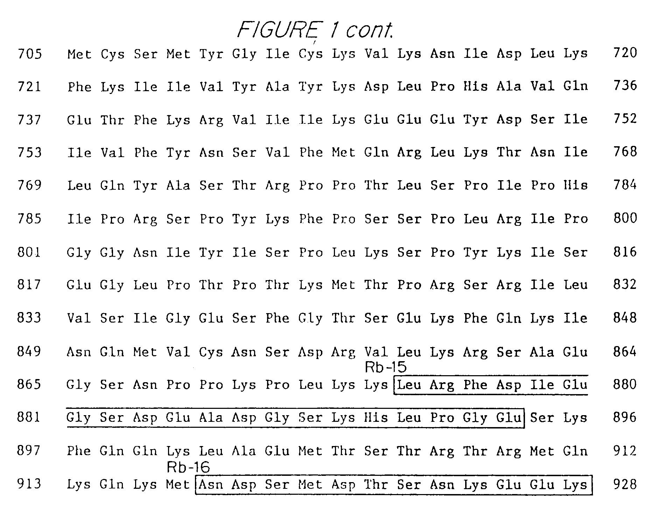

The method of selecting polypeptides of this invention utilized the reported cDNA sequences of the Rb gene and the 928 amino acid sequence deduced from the longest open reading frame of the Rb gene cDNA. The deduced amino acid sequence for the human Rb gene is shown in Fig. 1.

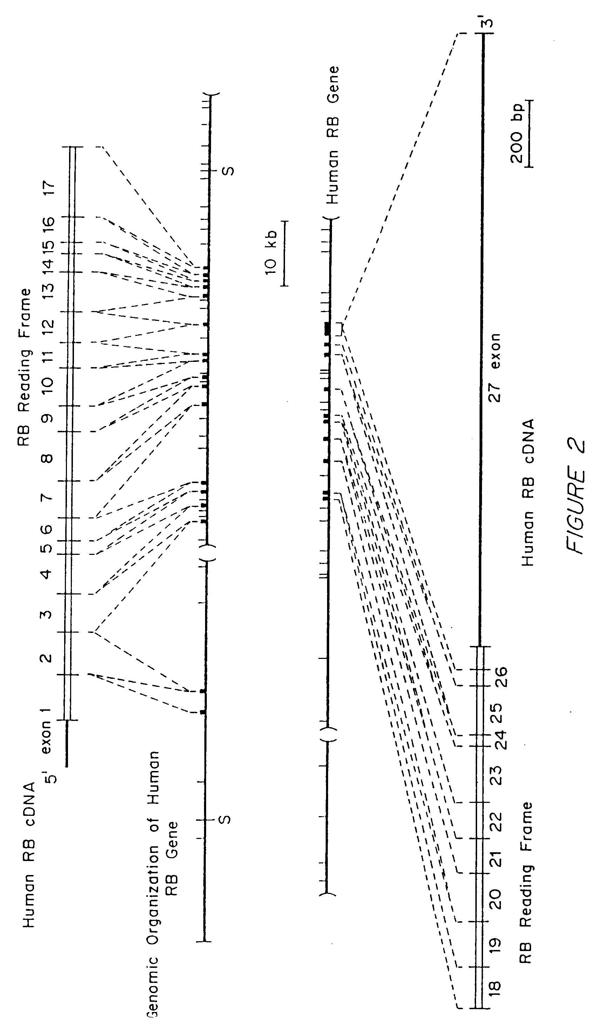

The Rb protein amino acid sequence and full cDNA sequence used in this invention was reported in Lee, W. H., Shew, J. Y., Hong, F. D., Sery, T. W., Donoso, L. A., Young, L. J., Bookstein, R., Lee, E. Y. H. "The retinoblastoma susceptibility gene encodes a nuclear phosphoprotein associated with DNA binding activity", Nature, Vol. 329, pp.642-645 (1987). Friend, S. H., Horowitz, J. M., Gerber, M. R., Wang, X. F. Bogenmann, E., Li, F. P., Weinberg, R. A. "Deletions of a DNA sequence in retinoblastoma and mesenchymal tumors: organization of the sequence and its encoded protein", Proc., Natl. Acad. Sci. U.S.A., Vol. 84, pp.9059-9063 (1987); "Human DNA in Diagnosis of Retinoblastoma", Mass. Eye & Ear Infirmary, EP-A2-0259,031; Fung, T'Ang, Murphree and Benedict, "A Method of Detecting the Predisposition to Retinoblastoma and a Method for Detecting a Retinoblastoma Gene Defect in Tumors Using a Retinoblastoma Gene Probe", Int'l. Publn. No. WO 88/09387, published December 1, 1988 (Int'l. Appln. No. PCT/US88/01809, which are incorporated by reference herein. A schematic Rb gene map for reference is shown in Figure 2 showing the location of the 27 exons.

The method of identification of the epitopes uses the entire amino acid sequence of the Rb protein and evaluates each consecutive sequence of at least 10 amino acids for solubility and hydrophilicity. The polypeptide must have a combination of low solubility and high hydrophilicity and thus have been defined as "flexible". The hydrophilic amino acids are thought to be more likely to be exposed on the surface of a native protein. The hydrophilic peptides traditionally have been preferred and are more likely to be soluble for peptide-carrier protein coupling reactions. Therefore, high solubility was considered an advantage in selecting antigenic polypeptide. However, the antigens of this invention have low solubility with high hydrophilicity.

The solubility characteristics of individual amino acids used in this invention are shown in TABLE 1 below.

4

TABLE 1

| AMINO ACID SOLUBILITY | | |
|---|---|---|
| Grade I insoluble solubility <1g/100ml $H_2O$ | Grade II slightly soluble solubility = 1-5g/100ml $H_2O$ | Grade III soluble solubility >5g/100ml $H_2O$ |
| Asp<br>Glu<br>Tyr | Asn<br>Gln<br>His<br>Ile<br>Leu<br>Phe<br>Trp | Ala<br>Arg<br>Cys<br>Gly<br>Lys<br>Met<br>Pro<br>Ser<br>Thr<br>Val |

The synthetic polypeptides of this invention must have low solubility characteristics as defined herein. Based on solubility characteristics, the amino acids are broken down into three categories shown in TABLE I including grade I-insoluble, solubility <1g/100ml $H_2O$; grade II-slightly soluble, solubility = 1-5g/100ml $H_2O$; and grade III-soluble, solubility >5g/100ml $H_2O$. Low solubility is characterized by taking the solubility grade of each amino acid in a sequence and selecting sequences of greater than 10 amino acids in which the number of grade I (insoluble) and grade II (slightly soluble) amino acids is greater than or equal to the number of grade III (soluble) amino acids in the sequence, preferably, the number of grade I amino acids is greater than or equal to the number of grade III amino acids in the sequence.

In addition to the analysis of the sequences for the desired low solubility profile, a hydrophilicity determination of the sequence is made. For the purposes of this invention, hydrophilicity values of Hopp and Woods are used as described in "Prediction of protein antigenic determinants from amino acid sequences" Proc. Natl. Acad. Sci., Vol. 78, pp 3824-3828 (1981). The average hydrophilicity of the amino acid sequence must be greater than 0.75.

The hydrophilicity values of the various amino acids used in this invention are presented in the following TABLE 2.

TABLE 2

| AMINO ACID HYDROPHILICITY VALUE* | |
|---|---|
| Amino Acid | Hydrophilicity Value |
| Arg | 3.0 |
| Asp | 3.0 |
| Glu | 3.0 |
| Lys | 3.0 |
| Ser | 0.3 |
| Asn | 0.2 |
| Gln | 0.2 |
| Gly | 0.0 |
| Pro | 0.0 |
| Thr | -0.4 |
| Ala | -0.5 |
| His | -0.5 |
| Cys | -1.0 |
| Met | -1.3 |
| Val | -1.5 |
| Ile | -1.8 |
| Leu | -1.8 |
| Tyr | -2.3 |
| Phe | -2.5 |
| Try | -3.4 |

* Hopp and Woods (1981)

A peptide of at least 10 amino acids is identified using the above method. The peptide can be extended by adding amino acids corresponding to the protein sequence to either end of the 10 amino acid sequence in a symmetrical or an asymmetrical manner, maintaining the initial peptide sequence.

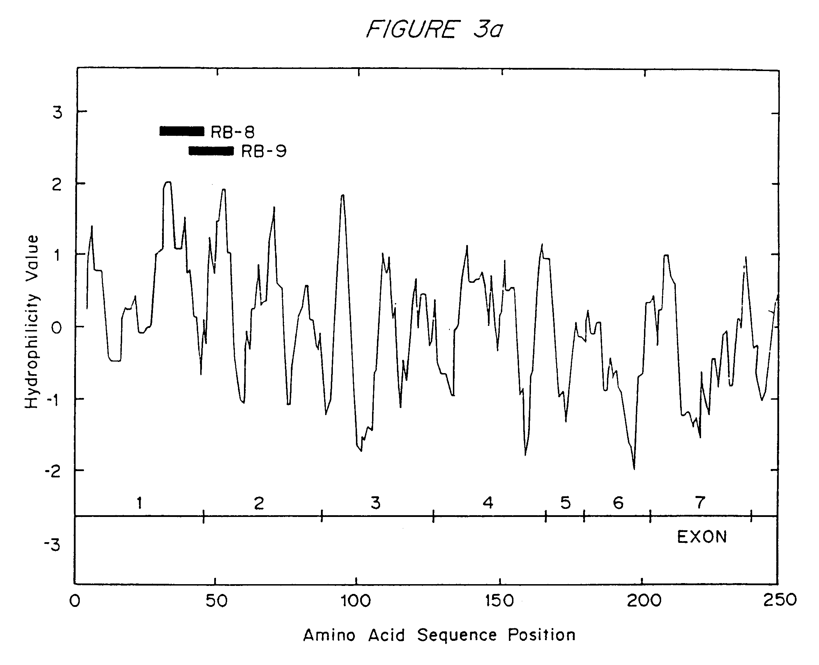

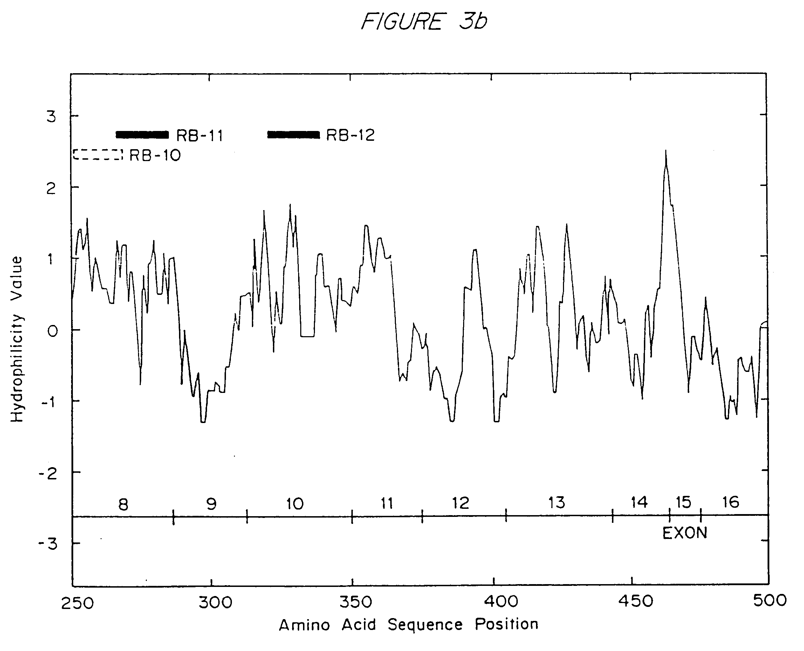

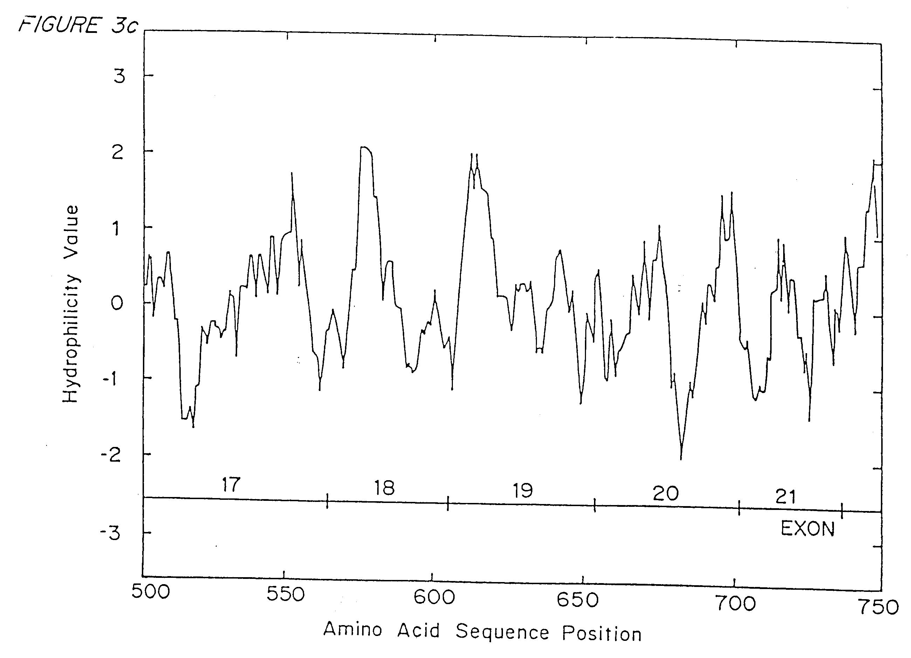

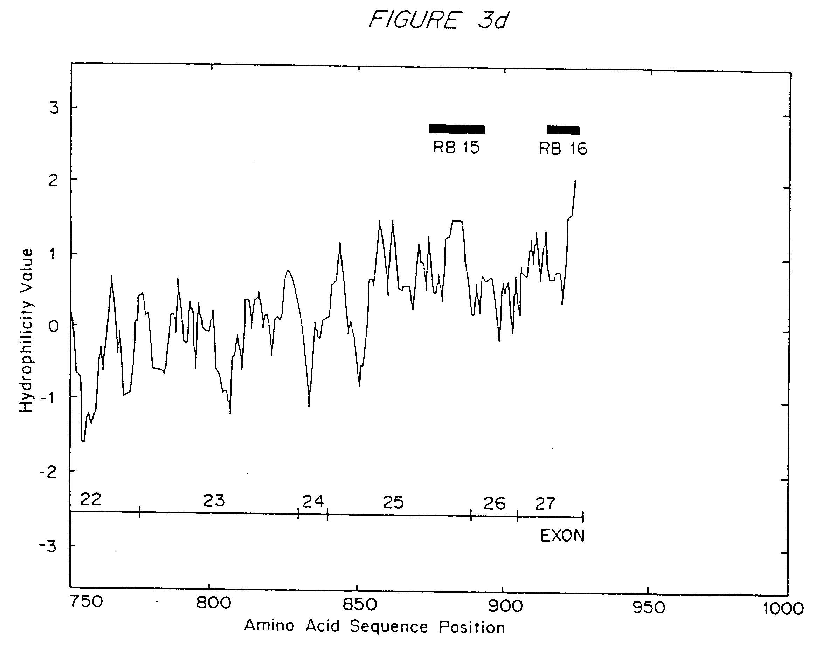

Figure 3 (composed of Figs. 3a, 3b, 3c and 3d) is a hydrophilicity profile of the Rb protein. At least six confirmed Rb antigens, designated as Rb-8, Rb-9, Rb-11, Rb-12, Rb-15 and Rb-16 have been selected by the method of this invention and are shown by the dark bars on Fig. 3 over relative placement of the exons of the Rb gene. A polypeptide indicated by reference numeral Rb-10 and a dotted bar was prepared contrary to the flexibility criteria of this invention. The average hydrophilicity of Rb-10 is 0.86 (>0.75), but the ratio between insoluble, slightly soluble and soluble amino acid residues of Rb-10 is 1:5:14 with the number of grade I and grade II amino acids being less than the number of grade III amino acids. Rb-10 is extremely soluble and deliquescent in air. The polypeptide was used for immunization with and without a carrier protein. After processing the antisera via the same multistage purification steps of this invention no Rb protein was detected in immunoprecipitation or Western immunoblotting using the anti-Rb-10 antibody.

The preferred antigen is Rb-12. Also preferred as antigens are analogues to Rb-12 wherein at least 80% amino acid homology with the Rb-12 sequence is retained in the analogue. The other peptides can be varied in the same manner with at least 80% amino acid homology.

The following TABLE 3 includes the complete sequences of the Rb antigens referenced in Fig. 2. The antigens are expected to produce a highly specific anti-Rb antibody.

6

### TABLE 3
### Rb PROTEIN ANTIGEN PEPTIDES

| Name | Amino acid sequence | Solubility | | | Average |
|------|---------------------|---|---|---|---------|
| | | I | II | III | Hydrophilicity |
| Rb-8 | Glu-Glu-Asp-Pro-Glu-Gln-Asp-Ser-Gly-Pro-Glu-Asp-Leu-Pro-Leu-Val-Arg | 7 | 3 | 7 | 1.14 |
| Rb-9 | Glu-Asp-Leu-Pro-Leu-Val-Arg-Leu-Glu-Phe-Glu-Glu-Thr-Glu-Glu-Pro-Asp-Phe | 8 | 5 | 5 | 0.82 |
| Rb-11 | Glu-Asn-Asp-Thr-Arg-Ile-Ile-Glu-Val-Leu-Cys-Lys-Glu-His-Glu-Cys-Asn-Ile-Asp-Glu | 7 | 6 | 7 | 0.79 |
| Rb-12 | Glu-Glu-Ile-Tyr-Leu-Lys-Asn-Lys-Asp-Leu-Asp-Ala-Arg-Leu-Phe-Leu-Asp-His-Asp-Lys | 7 | 8 | 5 | 0.77 |
| Rb-15 | Leu-Arg-Phe-Asp-Ile-Glu-Gly-Ser-Asp-Glu-Ala-Asp-Gly-Ser-Lys-His-Leu-Pro-Gly-Glu | 6 | 5 | 9 | 0.79 |
| Rb-16 | Asn-Asp-Ser-Met-Asp-Thr-Ser-Asn-Lys-Glu-Glu-Lys | 4 | 2 | 6 | 1.44 |

The preferred antigen included in TABLE 3 is Rb-12. The method of antigen sequence identification can be used to determine other synthetic polypeptide antigens in addition to those specifically referred to in TABLE 3.

A high concentration of an individual peptide without conjugating to a carrier protein was injected into rabbits. Peptide antibodies were often present in high titer after the third or fourth boost. The peptides may also be conjugated to a carrier protein and produce highly specific antibodies.

In addition to the Rb antigenic amino acid sequence described above, a modification can be made to the polypeptides without decreasing their effectiveness to obtain a pure highly specific anti-Rb antibody. An additional amino acid may be added to the end of the sequence for conjugation with a carrier protein. For example, an amino terminal cysteine was added to Rb-12, conjugated to Keyhole Limpet Hemocyanin (KLH) and used for immunization. A purified high-affinity monospecific anti-Rb antibody was obtained.

The procedures described were used to produce the highly specific anti-Rb antibodies. The antibodies were then used for immunoprecipitation, Western Blot and other processes. The results reported herein follow the described procedures with the express modifications from typical protocol. However, the antibodies may be produced and purified by other procedures or modifications of procedures in addition to those reported herein which can be practised by those skilled in the art.

The procedure to produce anti-Rb antibody IgGs starts with the identification of the peptide or peptides with or without a carrier protein to be used. (See TABLE 3 and discussion.) Rabbits are immunized with an initial intramuscular injection of 10 mg peptide in complete Freud's adjuvant. Every two weeks there are boosts of 5-10 mg of peptide in incomplete Freud's adjuvant.

The rabbit antiserum is often present in a high titer after the third or fourth boost. The rabbit serum immune globulins were precipitated with ammonium sulfate, typically 45% ammonium sulfate at 4°C for approximately 12 hours. After precipitation, the crude immune globulin pellet was resuspended in PBS.

The crude immune globulin solution was desalinized using dialysis method. There were 3 changes of 17.5 mM phosphate buffer of pH 6.3 over about 48 hours. The rabbit IgGs were then isolated by ion exchange chromatography. In the chromatography, "Sephacel" and "Sepharose" materials are used. These words are Registered Trade Marks of Pharmacia. DEAE-Sephacel was pre-equilibrated with 17.5 mM phosphate buffer at pH 6.3. The IgG fractions were eluted with 17.5 mM phosphate buffer at pH 6.3.

The IgG fraction was then subjected to affinity chromatography. The pH was adjusted to 7.2-7.4 by adding 1N NaOH. The pure anti-RB IgGs were eluted from peptide CNBr-Sepharose columns with 0.1M of glycine buffer (pH gradient 3.5-2.2) at a low pH range of pH 2.2-2.4. Typically, the lowest pH used for immuno affinity purification procedures of antibodies with 0.1M glycine is pH 2.5. The purification method used in this invention is unique. The use of a high concentrate of salts in place of 0.1M glycine will shift the pH range for elution. The preferred procedure is to absorb the IgGs to the immunoabsorbent by rotating at 4°C for approximately 12 hours. The immunoabsorbent is a CNBr-activated Sepharose (Pharmacia) conjugated with the relevant Rb peptides. The antibodies were then eluted from the column with 0.1 M glycine and a pH gradient 3.6 to 2.2 and the anti-Rb antibodies collected at the low pH range of 2.4-2.2 The pure high-affinity IgGs immunoprecipitated Rb proteins at increased washing stringencies.

For immunoprecipitation, the immune complex produced by this method was collected on Protein A-Sepharose CL-4B beads (Sigma) suspension which was made immediately prior to use. (To make a 2% Protein A-sepharose solution, the Protein A-sepharose CL-4B beads were suspended with PBS and rocked for one hour at 4°C. The beads were centrifuged at 8,000 rpm for five minutes at 4° in a microcentrifuge. The pellet was washed with PBS and rocked for five minutes and then spun at 8,000 rpm in a microcentrifuge for five minutes at 4°C. This washing and centrifugation process was repeated three to five times. After the last centrifugation in this step, the pellet was resuspended in 1ml with resuspending buffer to give a 2% solution. A sample of 10ml of the resuspending buffer contains 5ml PBS, 50$\mu$l 10% NaN$_3$, 0.4g BSA (Bovine Serum Albumin), plus 5 ml of 200mM NaCl, 20mM Tris-HCl pH7.4 and 0.4% NP-40 detergent. The buffer was filtered through a 0.45$\mu$m filter.) The Protein A-sepharose bead's suspension can be used immediately or within 24 hours after preparation.

The cell culture to be examined for the presence of Rb proteins was grown in tissue culture medium such as DMEM containing 10% FCS or other tissue culture medium. The cells to be used for typical Rb gene product pattern identification should be in the log. phase. For the purpose of this description, the process will be described using 1-2 x 10$^6$ cells. The cells were starved in media lacking methionine, such as -met MEM 10% dialyzed FCS media, for one to one and one-half hours. [$^{35}$S]-Methionine 250$\mu$ci/1.3ml media was added to the cells in 60mm dish for three to four hours. The cells were washed once with PBS. A lysis buffer of 60mM NaCl, 30mM Tris-HCl pH7.4, 0.2% NP-40, 0.5% Na-deoxycholate, and 0.1% SDS was prepared. Just before using the lysis buffer 0.05mg/ml aprotinin was added. In this protocol, 0.6ml lysis buffer/dish of cells at 4°C was added for 20 minutes. The cells were then disrupted by repeated aspiration through a 21 gauge needle. After lysis and aspiration, the cell lysate was centrifuged at 40,000 rpm for 22 minutes to remove the cell debris. The supernatant containing the protein was about 0.6ml. The supernatant was mixed with 0.4ml PBS containing 0.05mg/ml aprotinin. One $\mu$l of preimmune rabbit sera was added and the mixture rocked for two hours at about 4°C.

Ten percent Protein A-S.aureus cells were also prepared. One gram of reconstituted Protein A-positive S.aureus cells (BMB) with 10ml double distilled H$_2$0 was aliquoted in 1ml or smaller volumes and frozen at -80°C. Immediately before use in the immunoprecipitation procedure, the aliquot was thawed and microcentrifuged at 2,500 rpm for two minutes. The pellet was resuspended in resuspending buffer (described above) of the same volume. The solution contained S.aureus cells at 10%. Ten $\mu$l of 10% Protein A-S.aureus cells were added to the cell lysate and rocked for 30 minutes at 4°C. The mixture was then centrifuged in a microfuge at 13,000 rpm for five minutes at 4°C to remove the precipitated nonspecific immune complexes and IgGs. An additional 10$\mu$l of 10% Protein A-S.aureus was added to the supernatant and rocked for one hour at 4°C. The mixture was microcentrifuged at 13,000 rpm for 15 minutes at 4°C to remove any additional precipitated nonspecific immune complexes and IgGs.

The remaining supernatant was then ready for the addition of the anti-Rb antibodies. In the purification procedure previously described, it has been found that using the buffer system described herein the high affinity anti-Rb antibodies are collected at a low pH range of 2.4 to 2.2. It has been found that the more highly specific antibodies within the 2.2 to 2.4 pH range are not necessarily eluted during the protein peak.

Each fraction collected in the pH gradients needs to be tested to determine the fraction with the highly specific high affinity Rb antibodies, because in some cases, it has been found that the antibodies of choice are not in the highest peak fraction eluted from the immunoabsorbent.

From a selected fraction 0.1$\mu$g/10$\mu$l of anti-Rb-IgG containing 1% BSA was added to the supernatant and rocked at least two hours at 4°C. Fifty $\mu$l of the fresh 2% Protein A-sepharose was added and rocked for two hours at 4°C. The mixture was then centrifuged in a microfuge at 8,000 rpm for five minutes at 4°C. The Protein A-sepharose beads were washed with gradations of buffers from high salt concentration to low salt concentration to wash out any remaining nonspecific binding proteins that had remained with the Protein A-sepharose beads. The beads were washed with 1ml of HSW buffer 1M NaCl, 10mM Tris-HCl pH7.4, 0.2% NP-40 with the addition of 10ug/ml aprotinin, 0.5ug/ml leupeptin, 0.7$\mu$g/ml pepstatin added just before use. The beads were washed three times with the HSW buffer for five minutes each followed by centrifugation at 8,000 rpm for five minutes at 4°C. The beads were then washed with 1ml of LSW buffer two times for five minutes each at 8,000 rpm at 4°C. The LSW buffer was made of 100mM NaCl, 10mM Tris-HCl pH7.4, 0.2% NP-40 with 10ug/ml aprotinin, 0.5ug/ml leupeptin, 0.7ug/ml pepstatin added before use. The beads were then washed one time by rocking for five minutes with the washing buffer W. The W buffer was composed of 10mM Tris-HCl pH7.4, 0.2% NP-40 with 10$\mu$g/ml aprotinin, 0.5$\mu$g/ml leupeptin, and 0.7$\mu$g/ml pepstatin added just prior to use. After the last washing to remove the nonspecific proteins, the beads were centrifuged at 8,000 rpm for five minutes at 4°C. The Protein A-sepharose beads with Rb protein immune complex were then placed in 15$\mu$l of W buffer.

The proteins were solubilized with 15$\mu$l of 2xsample buffer of 0.125M Tris-HCl (pH6.8), 20% glycerol, 4% (W/V) SDS, 10% $\beta$-Mercaptoethanol and 0.005% bromophenol blue. The mixture was heated at 100°C for five minutes and then centrifuged at 13,000 rpm for 15 minutes at 4°C. Solubilized proteins were then separated by 8% SDS-PAGE. Fluorography was performed as described by Bonner et al, Eur. J. Biochem. Vol. 36, p.83-88 (1974).

Samples for immunoblotting were prepared as described for immunoprecipitation except that after electrophoresis, the proteins were electroblotted to Immobilon (Registered Trade Mark) PVDF membranes (Millipore). Western immunoblotting was carried out on solubilized Rb protein immune complex separated by 8% SDS-PAGE. The SDS-PAGE wet gel plate was subject to electroblotting at 30V, 0.1A for 12 hours and 60V, 0.21A for one hour. The blotting buffer was made of 192mM glycine, 25mM Tris, pH8.3 and 20% Methanol, 0.01% SDS. The transfer membrane was floated on Tris buffered saline (TBS) of 10mM Tris-HCl, pH8.0 and 150mM NaCl to evenly wet, then submerged and rinsed briefly. The excess protein binding sites were blocked by incubating the membrane in TBS containing 5% BSA with gentle shaking for about ten hours at room temperature. The blots were then processed by modification of the Proto Blot Western Blot AP system (Promega). A primary antibody was bound by replacing the blocking solution with TBST of 10mM Tris-HCl pH8.0, 150mM NaCl and 0.05% Tween20 ("Tween" is a Registered Trade Mark) containing 5% BSA and 0.05$\mu$g/ml, 20ml for a 10x15cm membrane of selected Rb antibody. The solution was incubated with the membrane at room temperature for about 12 hours with gentle shaking. The membrane was washed with gentle shaking in TBST three times for ten minutes each.

The second antibody was bound to the primary antibody on the membrane in TBST. The second antibody used in the Promega kit was an anti-rabbit IgG(Fc) alkaline phosphatase conjugate. The membrane was incubated in TBST containing the second antibody (1:3750 dilution) for an hour with gentle shaking. The color reaction was according to the Promega method. The membrane was blotted damp dry with filter paper and placed in the color development solution which was prepared by adding 66$\mu$l NBT (nitro blue tetrazolium) 50mg/ml in 70% dimethylformamide to 10ml alkaline phosphatase (AP) buffer of 100mM Tris HCl, pH 9.5, 100mM NaCl, 5mM $MgCl_2$, mixed and 33$\mu$l BCIP (5-bromo-4-chloro-3-indolyl-phosphate) 50mg/ml in dimethylformamide was added and mixed again. The solution was protected from strong light and used within an hour. The membrane was incubated with the solution for 15 minutes to four hours. The color development was stopped at the desired intensity, and the membrane was rinsed twice with double distilled water.

Example 1

Peptide Rb-12 from TABLE 2 was selected based on the flexibility criteria of the 20 amino acid residues of the predicted Rb protein. New Zealand white rabbits were injected intramuscularly with 10 mg of Rb-12. Additional boosts were made and the method of the invention was followed as previously described. The IgGs purified were high-affinity antibodies and immunoprecipitated Rb protein at increased washing stringencies. The anti-Rb antibody has been identified as Rb-WL-1.

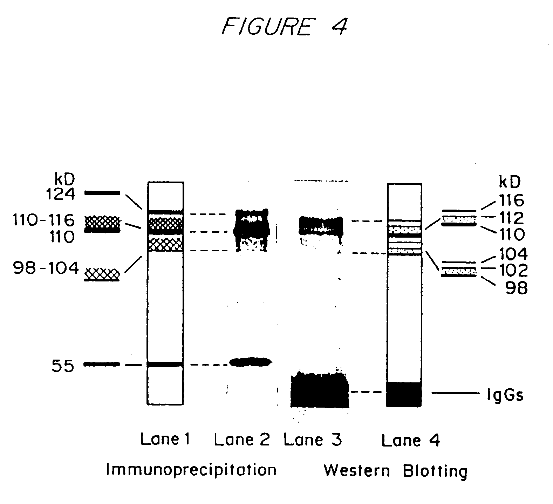

The Rb-WL-1 antibody immunoprecipitated Rb proteins from the $^{35}$S-labelled normal human fetal fibroblast cells WI38 in a specific pattern shown in Fig. 4; lane 1 is a schematic representation of lane 2. WS1 human fetal fibroblasts showed the same pattern. The normal fetal fibroblasts strains W1-38, WS1 were obtained from American Type Culture Collection. The pattern on SDS-PAGE consists of a lower band with a $M_r$ (apparent relative molecular mass) of 110kD which corresponds to the theoretical molecular mass expected from the Rb reading frame. The less distinct, variable region from 110kD to 116kD represents the Rb protein which is phosphorylated to a varying degree. The open reading frame of Rb cDNA sequence contains an in-frame second AUG codon located at nucleotides 475-477. (Lee et al. 1987) The protein initiated from the second AUG codon would be 12kD smaller than the first AUG codon. The immunoprecipitation of this invention after prolonged exposure reveals the band at 98kD which coincides exactly with the expected difference.

The phosphorylated Rb protein was confirmed by metabolically labeling with $^{32}$P-orthophosphate. The radioactive protein migrated as a thick band with a $M_r$ between 110kD and 116kD. No radioactivity was detected after dephosphorylation.

The immunoprecipitation also reflected proteins with $M_r$ of 124kD and 55kD. The Western Blot Fig. 4-lane 3 (schematically shown in lane 4) repeated all bands except for the 124kD and 55kD proteins. The 124kD and 55kD proteins were not detected by preimmuno serum, nor by antibody Rb-WL-1 that had been preabsorbed with excess peptide Rb-12. They are unlikely to be nonspecific contaminates. Since 124kD and 55kD proteins were not detected on Western Blot, the proteins did not share epitopes with the Rb protein and are likely to be stable complexes with the Rb protein.

The fibroblast W1-38 proteins detected with the Rb-WL-1 antibody produced a distinct Western blot pattern which identified the same Rb protein pattern from the immunoprecipitation. This normal Rb protein pattern on Western blots can be used as a standard for diagnostic work. The Rb-WL-1 produced a distinct Western Blot pattern in which the Rb proteins could be seen without extraneous bands.

The Rb gene is considered highly conserved. Rb-WL-1 has been confirmed to immunoprecipitate mouse Rb gene products. This antibody is likely to identify Rb protein in vertebrates including monkey, rat and chicken. Research on many species enhances the use of Rb-WL-1 and other products of this invention.

Example 2

An alternative embodiment for the flexible peptide which yields highly specific anti-Rb antibodies with the same quality as Example 1 has been obtained by adding an amino-terminal cysteine to peptide Rb-12 of TABLE 2. The peptide of this example was conjugated to Keyhole Limpet Hemocyanin (KLH) and used for immunization. Immunization with the KLH-Cys-Rb-12 of this example requires less peptide due to the coupling of the peptide with KLH. The procedure used KLH dialysed against 10 mM phosphate buffer at a pH of 7.2 at 4°C and adjusted to 20 mg/ml before use. The coupling procedure used 4 mg of KLH/200 μl with 5 mg of peptide, i.e., firstly 55 μl of 10 mM phosphate buffer were added to 4 mg/200 μl of KLH. Further 85 μl of m-maleimidobenzoyl N-hydroxysuccinimide ester (MBS) (3 mg/ml in dimethyl formamide) were added slowly. This mixture was stirred for 30 minutes at room temperature. The mixture was run through a G-25 Sephadex column and 1 ml fractions were collected. The fractions were analyzed for protein peaks and volume was recorded. Typically, there is 80% recovery of the KLH from the column and with this percentage of recovery 5 mg of peptide is added to the recovered KLH. The pH is adjusted to 7-7.5 and the mixture then stirred at room temperature for three hours. The conjugated peptide may be stored frozen.

The rabbits were immunized with KLH-Cys-Rb-12 and the same multi-stage purification procedure as described above in the specification produced highly specific anti-Rb antibodies. The results from immunoprecipitation and Western Blot gave the same or better identification of Rb gene products as described in Example 1.

Example 3

It is known that J82 bladder carcinoma cells have an exon 21 deletion in the Rb gene which produces a shortened Rb protein with an expected $M_r$ of 102.1kD. Horowitz, J. M. et al, "Point Mutational Inactivation of The Retinoblastoma Antioncogene", Science, Vol. 243, pp.937-940 (Feb. 1989) Also, cell cultures from K.G. retinoblastoma tumor cells have exon 14-17 deletions in the Rb gene, producing a shortened Rb protein with an expected $M_r$ of 90.4kD and Lowe retinoblastoma tumor cells have exon 21 plus exon 22 deletions of the Rb gene and produce a shortened Rb protein with an expected $M_r$ of 96.8kD.

Cell cultures were prepared from the three tumor cells and K. G. constitutional cells as well. The cells were metabolically labelled with $^{35}$S-Methionine. The results of the immunoprecipitation with Rb-WL-1 on SDS-PAGE comparing the normal WI38 human fibroblast Rb protein to the tumor proteins reflect the smaller apparent molecular mass of the abnormal Rb proteins relative to the exon deletion of the Rb gene.

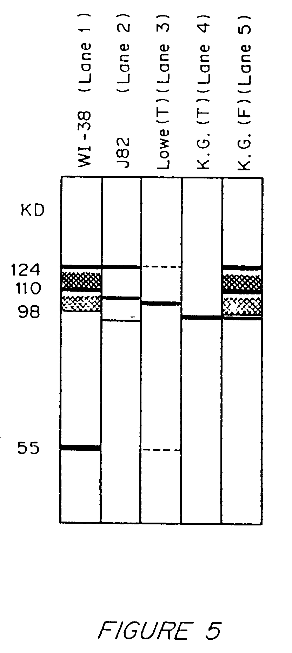

Fig. 5 is a schematic of the immunoprecipitation profile of the shortened proteins of this Example compared to normal Rb proteins. For example, the J82 protein was about 4kD smaller than the normal Rb protein shown at 110kD. (The 124 and 55kD proteins are not considered Rb proteins. See Example 1.) Likewise, the K.G. tumor Rb protein at 95kD and the Lowe tumor Rb protein at 101kD reflected lower molecular mass relative to the normal Rb protein based on the deletions of the exons and shortened mRNA. The measurement of molecular weight by SDS-PAGE does not give the exact molecular weight, due to secondary protein structure. However, the relative molecular weights are comparable by immunoprecipitation. The authenticity of the Rb-WL-1 antibody was evidenced by the immunoprecipitable proteins as the Rb gene products. The following TABLE 4 is a summary of the results of this example.

TABLE 4

| Rb-WL-1 DETECTABLE SHORTENED Rb PROTEIN IN TUMOR CELLS | | | | |
|---|---|---|---|---|
| Cells | Tumor | Structural Change in Rb Gene | Size of Rb Protein(kD) | |
| | | | $M_r$ Expected from Rb Reading Frame | Apparent $M_r$ on SDS-PAGE |
| WI38 | normal fibroblast (Lane 1) | - | 106.7 | 110 |
| J82 | bladder carcinoma (Lane 2) | exon 21 deletion | 102.1 | 106 |
| Lowe | retinoblastoma Lowe (T) (Lane 3) | exon 21 plus 22 deletion | 96.8 | 101 |
| K.G. | retinoblastoma (K.G. (T) (Lane 4) | exon 14-17 deletion | 90.4 | 95 |

Fig. 5 also shows in lane 5 the Rb immunoprecipitation profile of K.G. constitutional cells from the same individual with the truncated proteins from the K.G. retinoblastoma tumor shown in lane 4. The normal Rb protein pattern and the truncated protein pattern were both present. The individual with the hereditary form of Rb has both normal and abnormal Rb protein present indicative of a functioning normal Rb allele and a mutated allele as well. The method for detecting the presence of different Rb protein from tumor and constitutional cells further supports the diagnostic use of the invention for Rb gene products.

Example 4

One of the many uses of highly specific antibodies to gene products is use with a marker to detect the presence or absence of the gene product at the single cell level. Antibodies associated with stains have been used for immunohistochemical study for tissue culture as well as in vivo tissue sections from biopsies. The staining techniques utilizing antibodies are known to those skilled in the art.

The purified antibody Rb-WL-1 was used for immunostaining. Both cultured cell and tissue section staining was performed. The immunostaining with Rb-WL-1 in known Rb positive cell lines showed nuclear staining in proliferating cells and the absence of nuclear staining in resting cells including normal Rb + adult cells in vivo. The immunostaining in known Rb negative cell lines showed no staining in all cells. The Western immunoblotting technique confirmed the higher Rb protein content in the growing cells and a much reduced level in adult or resting cells. The highly sensitive antibody coupled with a stain is useful to determine Rb gene state in normal and cancer cells. The antibody was used to show location and cellular concentration of the Rb gene products.

Cell cultures tested for Rb positive and negative staining were prepared as follows. Adherent cells were grown on the sterile coverslips in the tissue culture dishes for a designed period. Suspension cells were first washed once with PBS after centrifugation and then resuspended in PBS. A drop of cells was added to the poly-L-lysine-coated coverslip and incubated for 20 minutes at room temperature. Cells on the coverslips were fixed in 45% acetone, 10% formaldehyde in 0.1 M phosphate buffer for 5 minutes. Fixed and permeabilized cells were washed in PBS with 6 changes over 15 minutes and then preincubated in 5% BSA, 4% normal goat serum in phosphate buffer in a moist chamber for 4 hours at room temperature. RB-WL-1 antibody was diluted 1:25 in 5% BSA, 4% normal goat serum and 0.02% Triton X-100 in phosphate buffer resulting in the final concentration of RB-WL-1 antibody to be 8 $\mu$g/ml and incubated with the cells overnight. After washing with 6 changes of Tris-buffered saline (TBS) containing 0.02% Triton X-100 over 15 minutes, the cells were labeled for 2 hours with goat anti-rabbit IgG-alkaline phosphatase conjugated antibody diluted 1:1000 in phosphate buffer containing 5% BSA, 4% normal goat serum and 0.02% Triton X-100. Cells were then washed with TBS containing 0.02% Triton X-100 for one hour, followed by incubation with substrates (bromochloroindolyl phosphate/nitro blue tetrazolium, BCIP/NBT, Promega). The reactions were stopped 7 to 10 minutes later by washing cells with double-distilled water. Cells were then viewed and photographed with an Olympus photomicroscope.

The immunostaining using a colloidal gold method was carried out basically as described above, except a goat anti-rabbit IgG-gold labeled antibody was used as the second antibody, followed by washing with PBS (3 x 10 minutes) and distilled water (3 x 3 minutes). The cells were then processed for silver enhancement using reagents named Intense M (manufactured by Janssen Biotech N.V. B-2430 Olen, Belgium).

For preparing frozen tissue sections, a small sample was fixed with freshly prepared 4% paraformaldehyde for at least 24 hours in 4°C. It was then immersed for 3 to 4 hours in 0.1 M sodium phosphate buffer, pH 7.4, containing 5% sucrose and for another 24 hours in 30% sucrose in the same phosphate buffer. The sample was frozen in liquid nitrogen and 6-$\mu$m-thick sections were cut in a cryostat at -20°C. Sections were thawed on poly-L-lysine-coated coverslips and washed once with PBS, and then fixed immediately for 5 minutes with 45% acetone, 10% formaldehyde in 0.1 M phosphate buffer. After rinsing sections in PBS, the tissue sections were processed for immunostaining using the alkaline phosphatase or colloidal gold method as described above. Finally, samples were counterstained with Harris hematoxylin-eosin Y (Sigma), dehydrated, mounted and examined.

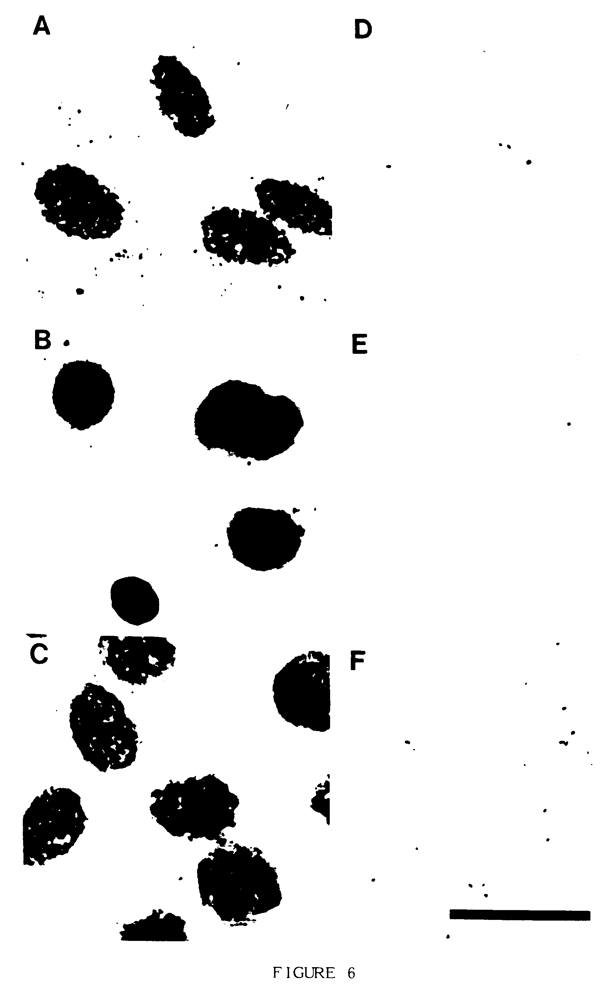

Figure 6 is a photograph of cell staining using Rb-WL-1 immunostaining. Figures 6A, B and C are known Rb+ positive cell lines fetal fibroblast W1-38, breast adenocarcinoma MCF7 and bladder carcinoma T24, respectively. The cells shown are in a proliferating state and evidence nuclear staining for the Rb gene product. Figures 6D and 6E are known Rb negative cell lines Fibrosarcoma Hs913T and retinoblastoma WER1-Rb-1 which show no staining. Figure 6F is the Rb positive cell line bladder carcinoma T24 processed for staining using RB-WL-1 antibody, which was preincubated with an excess of immunizing peptide RB-12 and the staining with the corresponding antibody Rb-WL-1 was blocked.

Immunostaining with Rb-WL-1 is another example of use of the highly specific antibody products developed by this invention. The use of antibodies is known to those skilled in the art. It is not intended to limit the coverage of this invention on any specific uses of the antibodies or the method of selection of antibodies.

**Claims**

1. A synthetic polypeptide for production of antibodies to retinoblastoma gene product proteins, said polypeptide being characterized by comprising a sequence of at least 10 amino acids of a protein predicted from said gene, by having over its entire sequence an average Hopp and Woods hydrophilicity greater than 0.75 and by having the number of grade I amino acids, namely Asp Glu or Tyr, alone or together with the number of Grade II amino acids, namely Asn, Gln, His, Ile, Leu, Phe or Trp, equal to or greater than the number of Grade III amino acids, namely Ala, Arg, Cys, Gly, Lys, Met, Pro, Ser, Thr or Val.

2. A synthetic polypeptide according to Claim 1, characterized in that the number of Grade I amino acids alone is equal to or greater than the number of Grade III amino acids.

3. A synthetic polypeptide according to Claim 1, characterized in that the number of Grade I and Grade II amino acids together is equal to or greater than the number of Grade III amino acids.

4. A synthetic polypeptide according to Claim 1 or 2, characterized in that it has sequence of amino acids:

```
Glu-Glu-Asp-Pro-Glu-Glu-Asp-Ser-Gly-Pro-Glu-Asp-Leu-Pro-
Leu-Val-Arg (Rb-8),


Glu-Asp-Leu-Pro-Leu-Val-Arg-Leu-Glu-Phe-Glu-Glu-Thr-Glu-
Glu-Pro-Asp-Phe (Rb-9),


Glu-Asn-Asp-Thr-Arg-Ile-Ile-Glu-Val-Leu-Cys-Lys-Glu-His-
Glu-Cys-Asn-Ile-Asp-Glu (Rb-11),


Glu-Glu-Ile-Tyr-Leu-Lys-Asn-Lys-Asp-Leu-Asp-Ala-Arg-Leu-
Phe-Leu-Asp-His-Asp-Lys (Rb-12),



Leu-Arg-Phe-Asp-Ile-Glu-Gly-Ser-Asp-Glu-Ala-Asp-Gly-Ser-
Lys-His-Leu-Pro-Gly-Glu (Rb-15) or


Asn-Asp-Ser-Met-Asp-Thr-Ser-Asn-Lys-Glu-Glu-Lys (Rb-16),
```

or a sequence which has at least 80 percent homology therewith.

5. A synthetic polypeptide according to Claim 4, characterized in that it further comprises an amino acid added to the end of the sequence for conjugation to a carrier protein.

6. A synthetic polypeptide according to Claim 4, characterized in that it has the sequence of amino acids of formula:

```
Cys-Glu-Glu-Ile-Tyr-Leu-Lys-Asn-Lys-Asp-Leu-Asp-Ala-Arg-
Leu-Phe-Leu-Asp-His-Asp-Lys (Cys-Rb-12)
```

7. An antigen comprising a synthetic polypeptide claimed in any preceding Claim, associated with a carrier protein.

8. An antigen according to Claim 7, characterized in that the synthetic polypeptide is conjugated to the carrier protein.

9. An antiserum to retinoblastoma gene products which is produced by a polypeptide claimed in any one of Claims 1 to 6 or by an antigen claimed in Claim 7 or 8.

10. An antibody which is immunospecific to retinoblastoma gene products and which is produced in response to a polypeptide claimed in any one of Claims 1 to 6 or to an antigen claimed in Claim 7 or 8.

11. An antibody according to Claim 10, which is of IgG.

**12.** An antibody according to Claim 11, which is monoclonal.

**13.** An antibody according to Claim 11, which is polyclonal.

**14.** An antibody according to Claim 10, 11, 12 or 13, associated with a marker for detection of the presence of the antibody.

**15.** An antibody according to Claim 14, characterized in that the marker is immunohistochemical.

**16.** Use of an antiserum claimed in Claim 9, or an antibody claimed in any one of Claims 10 to 15, to detect the presence or absence of retinoblastoma gene products.

**17.** Use of an antiserum claimed in Claim 9, or antibody claimed in any one of Claims 10 to 15 to a polypeptide having the Rb-12 sequence set forth in Claim 4 or Cys-Rb-12 sequence claimed in Claim 6 to detect abnormal retinobastoma gene products.

**Patentansprüche**

**1.** Synthetisches Polypeptid zur Bildung von Antikörpern gegen Retinoblastom-Genproduktproteine, wobei das Polypeptid durch die Zusammensetzung einer Sequenz von wenigstens 10 Aminosäuren eines Proteins, das von dem Gen vorherbestimmt wird, durch eine auf seiner gesamten Sequenz durchschnittliche Hopp and Woods Hydrophilie größer als 0,75 und durch das Vorhandensein einer Anzahl von Stufe I Aminosäuren, nämlich Asp, Glu oder Tyr, allein oder zusammen mit der Anzahl an Stufe II Aminosäuren, nämlich Asn, Gln, His, Ile, Leu, Phe oder Trp, gleich oder größer als die Anzahl an Stufe III Aminosäuren, nämlich Ala, Arg, Cys, Gly, Lys, Met, Pro, Ser, Thr oder Val gekennzeichnet ist.

**2.** Synthetisches Polypeptid nach Anspruch 1, bei dem die Anzahl der Stufe I Aminosäuren allein gleich oder größer ist als die Anzahl an Stufe III Aminosäuren.

**3.** Synthetisches Polypeptid nach Anspruch 1, bei dem die Anzahl an Stufe I und Stufe II Aminosäuren zusammen gleich oder größer ist als die Anzahl an Stufe III Aminosäuren.

**4.** Synthetisches Polypeptid nach den Ansprüchen 1 oder 2 mit folgender Sequenz von Aminosäuren:

```
Glu-Glu-Asp-Pro-Glu-Glu-Asp-Ser-Gly-Pro-Glu-Asp-Leu-Pro-Leu-
Val-Arg (Rb-8),

Glu-Asp-Leu-Pro-Leu-Val-Arg-Leu-Glu-Phe-Glu-Glu-Thr-Glu-Glu-
Pro-Asp-Phe (Rb-9),

Glu-Asn-Asp-Thr-Arg-Ile-Ile-Glu-Val-Leu-Cys-Lys-Glu-His-Glu-
Cys-Asn-Ile-Asp-Glu (Rb-11),
```

```
Glu-Glu-Ile-Tyr-Leu-Lys-Asn-Lys-Asp-Leu-Asp-Ala-Arg-Leu-Phe-
Leu-Asp-His-Asp-Lys (Rb-12),
```

```
Leu-Arg-Phe-Asp-Ile-Glu-Gly-Ser-Asp-Glu-Ala-Asp-Gly-Ser-Lys-
His-Leu-Pro-Gly-Glu (Rb-15) oder
```

```
Asn-Asp-Ser-Met-Asp-Thr-Ser-Asn-Lys-Glu-Glu-Lys (Rb-16),
```

oder mit einer Sequenz, die wenigstens zu 80 % hiermit homolog ist.

5. Synthetisches Polypeptid nach Anspruch 4, dadurch gekennzeichnet, daß es weiterhin aus einer Aminosäure besteht, die am Ende der Sequenz zur Konjugierung mit einem Carrier-Protein hinzugefügt wird.

6. Synthetisches Polypeptid nach Anspruch 4, dadurch gekennzeichnet, daß es die Sequenz von Aminosäuren folgender Formel besitzt:

```
Cys-Glu-Glu-Ile-Tyr-Leu-Lys-Asn-Lys-Asp-Leu-Asp-Ala-Arg-Leu-
Phe-Leu-Asp-His-Asp-Lys (Cys-Rb-12)
```

7. Antigen, bestehend aus einem synthetischen Polypeptid nach einem der vorhergehenden Ansprüche, verbunden mit einem Carrier-Protein.

8. Antigen nach Anspruch 7, dadurch gekennzeichnet, daß das synthetische Polypeptid mit dem Carrier-Protein konjugiert ist.

9. Antiserum gegen Retinoblastom-Genprodukte, welches durch ein Polypeptid, nach einem der Ansprüche 1 bis 6 oder durch ein Antigen nach den Ansprüchen 7 oder 8 gebildet wird.

10. Antikörper, der immunspezifisch zu den Retinoblastom-Genprodukten ist und der als Antwort auf ein Polypeptid, nach einem der Ansprüche 1 bis 6 oder auf ein Antigen nach den Ansprüchen 7 oder 8 gebildet wird.

11. Antikörper nach Anspruch 10, dadurch gekennzeichnet, daß er ein Immunglobulin G ist.

12. Antikörper nach Anspruch 11, dadurch gekennzeichnet, daß er monoklonal ist.

13. Antikörper nach Anspruch 11, dadurch gekennzeichnet, daß er polyklonal ist.

14. Antikörper nach Anspruch 10, 11, 12 oder 13, dadurch gekennzeichnet, daß er mit einem Markierer für die Identifizierung des Vorhandenseins des Antikörpers verbunden ist.

15. Antikörper nach Anspruch 14, dadurch gekennzeichnet, daß der Markierer immunhistochemisch ist.

16. Verwendung eines Antiserums nach Anspruch 9 oder eines Antikörpers nach einem der Ansprüche 10 bis 15 zur Identifizierung der Anwesenheit oder Abwesenheit von Retinoblastom-Genprodukten.

17. Verwendung eines Antiserums nach Anspruch 9 oder eines Antikörpers nach einem der Ansprüche 10 bis 15 für ein Polypeptid mit der Rb-12-Sequenz nach Anspruch 4 oder mit einer Cys-Rb-12-Sequenz nach Anspruch 6 zur Identifizierung von pathologischen Retinoblastom-Genprodukten.

**EP 0 467 998 B1**

**Revendications**

1. Polypeptide synthétique pour la production d'anticorps contre des protéines exprimées par le gène de rétinoblastomes, ledit polypeptide étant caractérisé en ce qu'il comprend une séquence d'au moins 10 acides aminés d'une protéine déduite dudit gène, en ayant sur sa séquence entière une hydrophilicité moyenne de Hopp et Woods supérieure à 0,75 et en ayant le nombre des acides aminés du jeu I, à savoir Asp Glu ou Tyr, seul ou ensemble, avec le nombre des acides aminés du jeu II, à savoir Asn, Gln, His, Ile, Leu, Phe ou Trp, égal ou supérieur au nombre des acides aminés du jeu III à savoir Ala, Arg, Cys, Gly, Lys, Met, Pro, Ser, Thr ou Val.

2. Polypeptide synthétique selon la revendication 1, caractérisé en ce que le nombre des acides aminés du jeu I seul est égal à ou supérieur au nombre des acides aminés du jeu III.

3. Polypeptide synthétique selon la revendication 1, caractérisé en ce que le nombre des acides aminés du jeu I et du jeu II ensemble sont égaux ou supérieurs au nombre des acides aminés du jeu III.

4. Polypeptide synthétique selon la revendication 1 ou 2, caractérisé en ce qu'il comporte une séquence d'acides aminés :

```
Gly-Glu-Asp-Pro-Glu-Glu-Asp-Ser-Gly-Pro-Glu-Asp-Leu-Pro-
Leu-Val-Arg (Rb-8),
Glu-Asp-Leu-Pro-Leu-Val-Arg-Leu-Glu-Phe-Glu-Glu-Thr-Glu-
Glu-Pro-Asp-Phe (Rb-9),
Glu- Asn-Asp-Thr-Arg-Ile-Ile-Glu-Val-Leu-Cys-Lys-Glu-His-
Glu-Cys-Asn-Ile-Asp-Glu (Rb-11),
Glu-Glu-Ile-Tyr-Leu-Lys-Asn-Lys-Asp-Leu-Asp-Ala-Arg-Leu-
Phe-Leu-Asp-His-Asp-Lys (Rb-12)
Leu-Arg-Phe-Asp-Ile-Glu-Gly-Ser-Asp-Glu-Ala-Asp-Gly-Ser-
Lys-His-Leu-Pro-Gly-Glu (Rb-15) ou
Asn-Asp-Ser-Met-Asp-Thr-Ser-Asn-Lys-Glu-Glu-Lys (Rb-16),
```

ou une séquence qui a au moins 80 pourcent d'homologie avec celle-ci.

5. Polypeptide synthétique selon la revendication 4, caractérisé en ce qu'il comporte de plus un acide aminé ajouté à l'extrémité de la séquence pour conjugaison à une protéine support.

6. Polypeptide synthétique selon la revendication 4, caractérisé en ce qu'il comporte la séquence d'acides aminés de formule :

```
Cys-Glu-Glu-Ile-Tyr-Leu-Lys-Asn-Lys-Asp-Leu-Asp-Ala-Arg-
Leu-Phe-Leu-Asp-His-Asp-Lys (Cys-Rb-12).
```

7. Antigène comprenant un polypeptide synthétique selon l'une quelconque des revendications précédentes associé à une protéine support.

8. Antigène selon la revendication 7, caractérisé en ce que le polypeptide synthétique est conjugué à la protéine support.

16

9. Antisérum dirigé contre les produits du gène de rétinoblastomes qui est produit par un polypeptide selon l'une quelconque des revendications 1 à 6 ou par un antigène selon la revendication 7 ou 8.

10. Anticorps qui est immunospécifique vis-à-vis de produits du gène de rétinoblastomes et qui est produit en réponse à un polypeptide selon l'une quelconque des revendications 1 à 6 ou à un antigène selon la revendication 7 ou 8.

11. Anticorps selon la revendication 10, qui est de la classe des IgG.

12. Anticorps selon la revendication 11, qui est monoclonal.

13. Anticorps selon la revendication 1, qui est polyclonal.

14. Anticorps selon la revendication 10, 11, 12 ou 13, associé à un marqueur pour la détection de la présence de l'anticorps.

15. Anticorps selon la revendication 14, caractérisé en ce que le marqueur est immunohistochimique.

16. Utilisation d'un antisérum selon la revendication 9, ou d'un anticorps selon l'une quelconque des revendications 10 à 15, afin de détecter la présence ou l'absence des produits du gène de rétinoblastomes.

17. Utilisation d'un antisérum selon la revendication 9, ou d'un anticorps selon l'une quelconque des revendications 10 à 15 contre un polypeptide ayant la séquence Rb-12 énoncée dans la revendication 4 ou la séquence Cys-Rb-12 selon la revendication 6 pour détecter des produits anormaux du gène de rétinoblastomes.

## FIGURE 1

### The Complete Amino Acid Sequence of RB Protein

```
1    Met Pro Pro Lys Thr Pro Arg Lys Thr Ala Ala Thr Ala Ala Ala Ala    16
                                                      Rb-8
17   Ala Ala Glu Pro Pro Ala Pro Pro Pro Pro Pro Pro Glu Glu Asp         32
                               Rb-9
33   Pro Glu Gln Asp Ser Gly Pro Glu Asp Leu Pro Leu Val Arg Leu Glu     48

49   Phe Glu Glu Thr Glu Glu Pro Asp Phe Thr Ala Leu Cys Gln Lys Leu     64

65   Lys Ile Asp Asp His Val Arg Glu Arg Ala Trp Leu Thr Trp Glu Lys     80

81   Val Ser Ser Val Asp Gly Val Leu Gly Gly Tyr Ile Gln Lys Lys Lys     96

97   Glu Leu Trp Gly Ile Cys Ile Phe Ile Ala Ala Val Asp Leu Asp Glu    112

113  Met Ser Phe Thr Phe Thr Glu Leu Gln Lys Asn Ile Glu Ile Ser Val    128

129  His Lys Phe Phe Asn Leu Leu Lys Glu Ile Asp Thr Ser Thr Lys Val    144

145  Asp Met Ala Met Ser Arg Leu Leu Lys Lys Tyr Asp Val Leu Phe Ala    160

161  Leu Phe Ser Lys Leu Glu Arg Thr Cys Glu Leu Ile Tyr Leu Thr Gln    176

177  Pro Ser Ser Ser Ile Ser Thr Glu Ile Asn Ser Ala Leu Val Leu Lys    192

193  Val Ser Trp Ile Thr Phe Leu Leu Ala Lys Gly Glu Val Leu Gln Met    208

209  Glu Asp Asp Leu Val Ile Ser Phe Gln Leu Met Leu Cys Val Leu Asp    224

225  Tyr Phe Ile Lys Leu Ser Pro Pro Met Leu Leu Lys Glu Pro Tyr Lys    240

241  Thr Ala Val Ile Pro Ile Asn Gly Ser Pro Arg Thr Pro Arg Arg Gly    256
                                             Rb-11
257  Gln Asn Arg Ser Ala Arg Ile Ala Lys Gln Leu Glu Asn Asp Thr Arg    272

273  Ile Ile Glu Val Leu Cys Lys Glu His Glu Cys Asn Ile Asp Glu Val    288

289  Lys Asn Val Tyr Phe Lys Asn Phe Ile Pro Phe Met Asn Ser Leu Gly    304

305  Leu Val Thr Ser Asn Gly Leu Pro Glu Val Glu Asn Leu Ser Lys Arg    320
     Rb-12
321  Tyr Glu Glu Ile Tyr Leu Lys Asn Lys Asp Leu Asp Ala Arg Leu Phe    336

337  Leu Asp His Asp Lys Thr Leu Gln Thr Asp Ser Ile Asp Ser Phe Glu    352
```

18

## FIGURE 1 cont.

```
353   Thr Gln Arg Thr Pro Arg Lys Ser Asn Leu Asp Glu Glu Val Asn Val   368

369   Ile Pro Pro His Thr Pro Val Arg Thr Val Met Asn Thr Ile Gln Gln   384

385   Leu Met Met Ile Leu Asn Ser Ala Ser Asp His Pro Ser Glu Asn Leu   400

401   Ile Ser Tyr Phe Asn Asn Cys Thr Val Asn Pro Lys Glu Ser Ile Leu   416

417   Lys Arg Val Lys Asp Ile Gly Tyr Ile Phe Lys Glu Lys Phe Ala Lys   432

433   Ala Val Gly Gln Gly Cys Val Glu Ile Gly Ser Gln Arg Tyr Lys Leu   448

449   Gly Val Arg Leu Tyr Tyr Arg Val Met Glu Ser Met Leu Lys Ser Glu   464

465   Glu Glu Arg Leu Ser Ile Gln Asn Phe Ser Lys Leu Leu Asn Asp Asn   480

481   Ile Phe His Met Ser Leu Leu Ala Cys Ala Leu Glu Val Val Met Ala   496

497   Thr Tyr Ser Arg Ser Thr Ser Gln Asn Leu Asp Ser Gly Thr Asp Leu   512

513   Ser Phe Pro Trp Ile Leu Asn Val Leu Asn Leu Lys Ala Phe Asp Phe   528

529   Tyr Lys Val Ile Glu Ser Phe Ile Lys Ala Glu Gly Asn Leu Thr Arg   544

545   Glu Met Ile Lys His Leu Glu Arg Cys Glu His Arg Ile Met Glu Ser   560

561   Leu Ala Trp Leu Ser Asp Ser Pro Leu Phe Asp Leu Ile Lys Gln Ser   576

577   Lys Asp Arg Glu Gly Pro Thr Asp His Leu Glu Ser Ala Cys Pro Leu   592

593   Asn Leu Pro Leu Gln Asn Asn His Thr Ala Ala Asp Met Tyr Leu Ser   608

609   Pro Val Arg Ser Pro Lys Lys Lys Gly Ser Thr Thr Arg Val Asn Ser   624

625   Thr Ala Asn Ala Glu Thr Gln Ala Thr Ser Ala Phe Gln Thr Gln Lys   640

641   Pro Leu Lys Ser Thr Ser Leu Ser Leu Phe Tyr Lys Lys Val Tyr Arg   656

657   Leu Ala Tyr Leu Arg Leu Asn Thr Leu Cys Glu Arg Leu Leu Ser Gln   672

673   His Pro Glu Leu Glu His Ile Ile Trp Thr Leu Phe Gln His Thr Leu   688

689   Gln Asn Glu Tyr Glu Leu Met Arg Asp Arg His Leu Asp Gln Ile Met   704
```

## FIGURE 1 cont.

705 Met Cys Ser Met Tyr Gly Ile Cys Lys Val Lys Asn Ile Asp Leu Lys 720

721 Phe Lys Ile Ile Val Tyr Ala Tyr Lys Asp Leu Pro His Ala Val Gln 736

737 Glu Thr Phe Lys Arg Val Ile Ile Lys Glu Glu Glu Tyr Asp Ser Ile 752

753 Ile Val Phe Tyr Asn Ser Val Phe Met Gln Arg Leu Lys Thr Asn Ile 768

769 Leu Gln Tyr Ala Ser Thr Arg Pro Pro Thr Leu Ser Pro Ile Pro His 784

785 Ile Pro Arg Ser Pro Tyr Lys Phe Pro Ser Ser Pro Leu Arg Ile Pro 800

801 Gly Gly Asn Ile Tyr Ile Ser Pro Leu Lys Ser Pro Tyr Lys Ile Ser 816

817 Glu Gly Leu Pro Thr Pro Thr Lys Met Thr Pro Arg Ser Arg Ile Leu 832

833 Val Ser Ile Gly Glu Ser Phe Gly Thr Ser Glu Lys Phe Gln Lys Ile 848

849 Asn Gln Met Val Cys Asn Ser Asp Arg Val Leu Lys Arg Ser Ala Glu 864

Rb-15

865 Gly Ser Asn Pro Pro Lys Pro Leu Lys Lys Leu Arg Phe Asp Ile Glu 880

881 Gly Ser Asp Glu Ala Asp Gly Ser Lys His Leu Pro Gly Glu Ser Lys 896

897 Phe Gln Gln Lys Leu Ala Glu Met Thr Ser Thr Arg Thr Arg Met Gln 912

Rb-16

913 Lys Gln Lys Met Asn Asp Ser Met Asp Thr Ser Asn Lys Glu Glu Lys 928

FIGURE 2

FIGURE 3a

EP 0 467 998 B1

FIGURE 3b

FIGURE 3c

EP 0 467 998 B1

EP 0 467 998 B1

## FIGURE 3d

FIGURE 4

FIGURE 5

A

D

B

E

C

F

FIGURE 6